# EUROPEAN PATENT APPLICATION

(11) **EP 4 435 089 A1**
(43) Date of publication of application: **25.09.2024**
(21) Application number: 22896027.4
(22) Date of filing: 15.11.2022
(51) Int. Cl.: C12N 1/14, C12P 13/04, A23L 29/00, A23J 1/00, C12R 1/69

(54) **ASPERGILLUS ORYZAE STRAIN PRODUCING MYCELIUM INCLUDING ALL KINDS OF ESSENTIAL AMINO ACIDS**

(30) Priority: 16.11.2021 KR 20210157491
(71) Applicant: CJ CheilJedang Corporation, Seoul 04560 (KR)
(72) Inventor: LEE, Ja Young, Seoul 04560 (KR); JEON, Kyung Chan, Seoul 04560 (KR); KIM, Hae Bom, Seoul 04560 (KR); JANG, Ki Joo, Seoul 04560 (KR); VITON, Florian, Seoul 04560 (KR)
(74) Representative: Cabinet Laurent & Charras
(86) International application number: PCT/KR2022/018007
(87) International publication number: WO 2023/090821

(57) **Abstract**

The present application relates to a novel *Aspergillus oryzae* CJ11100 strain and a mycelium produced therefrom. Using the novel *Aspergillus oryzae* CJI1100 strain of the present application, a mycelium including all the kinds of essential amino acids can be mass-produced. The mycelium of the present application has, as a complete protein, the advantage of being used in various foods, including meat substitutes.

## Description

### BACKGROUND

### (a) Technical Field

The present application relates to a novel *Aspergillus oryzae* strain and mycelium produced therefrom. Further, it relates to a method for mass-producing mycelium from *Aspergillus oryzae* strain, and a food using the mycelium produced by the method.

### (b) Background Art

In a situation where awareness of food security due to environmental problems and population growth is increasing, interest in alternative foods is increasing in consideration of sustainability. In addition, as interest in health has recently increased, the demand for high-protein, low-calorie or low-fat foods has increased, and therefore, the market size for meat substitutes and alternative proteins is expected to further increase. In this situation, vegetable protein is being used as a protein material to replace meat obtained from livestock farming. Among them, vegetable meat using protein-rich soybeans is mainly used, but soybean vegetable meat has limitations in reproducing the unique texture of meat even if it has the appearance of meat through an extrusion molding process. As a vegetable protein material other than soybean, the use of mycelia of mushrooms with a fibrous structure is being reviewed, but there is a limitation that the protein content of mushroom mycelium is relatively low compared to soybean protein. Therefore, it is necessary to develop other protein materials with high protein content for meat substitute.

Fungi have long been used as a food ingredient for the production of a variety of foods and beverages, including fermented foods and alcoholic beverages. When culturing fungi in a liquid medium, only mycelium produced by the fungus can be obtained without forming spores. The fungus mycelium obtained through this culture method has a high protein content and a fibrous structure, which is advantageous for creating a texture of meat. In addition, since mycelium produced by fungi has a high content of glutamic acid, it has a savory taste and is tasteless, so it can be used as a variety of products added to food. Moreover, mycelium produced by fungi is rich in dietary fiber and is known to be effective in reducing blood cholesterol.

Korean Patent No. 10-1760184 suggests a method for increasing the glyceollin I content of soybeans by culturing after inoculating soybeans with mycelium of *Aspergillus sojae,* an edible fungal strain. Korean Patent No. 10-1929346 suggests a method for producing malted rice using *Aspergillus oryzae* strain, but it does not disclose a method for obtaining mycelium with excellent amino acid composition by culturing fungal strain. Accordingly, the inventors of the present application confirmed that the novel *Aspergillus* strain derived from fermented soybean lump produced mycelium composed of all kinds of essential amino acids and completed the present application.

### [Prior Art Documents]

### [Patent Documents]

(Patent Document 1) Korean Patent No.10-1760184
(Patent Document 2) Korean Patent No.10-1929346

### SUMMARY OF THE INVENTION

It is an object of the present application to provide a novel *Aspergillus oryzae* strain that produces mycelium comprising methionine.

Further, the present application is objected to provide mycelium produced from the strain.

Further, the present application is objected to provide a method for producing the mycelium from the strain.

Further, the present application is objected to provide a food containing the mycelium produced from the strain.

One aspect of the present application provides *Aspergillus oryzae* CJ11100 strain (Accession Number: KCCM13053P) that produces mycelium having an amino acid composition comprising methionine.

Another aspect of the present application provides mycelium produced from the *Aspergillus oryzae* CJI1100 strain.

Another aspect of the present application provides a method for producing mycelium comprising the following steps of: producing mycelium by culturing *Aspergillus oryzae* CJI1100 strain in a medium; and recovering the mycelium.

Another aspect of the present application provides a food containing the mycelium produced from the *Aspergillus oryzae* CJI1100 strain.

Hereinafter, the present application will be described in detail.

One aspect of the present application provides *Aspergillus oryzae* CJ11100 strain (Accession Number: KCCM13053P) that produces mycelium having an amino acid composition comprising methionine.

The *Aspergillus oryzae* CJ11100 strain of the present application is a novel strain isolated from traditional fermented soybean lump, and has the characteristics of producing mycelium containing all kinds of essential amino acids including methionine.

The strain of the present application was deposited with the Korean Culture Center of Microorganisms (KCCM) on October 05, 2021 and received the Accession number of KCCM13053P.

In one embodiment, the amino acid composition of the mycelium produced by the *Aspergillus oryzae* CJI1100 strain of the present application comprises methionine.

In one embodiment, the amino acid composition of the mycelium produced by the *Aspergillus oryzae* CJI1100 strain of the present application may further comprise at least one amino acid selected from the group consisting of lysine, threonine, valine, isoleucine, leucine, phenylalanine, tryptophan and histidine.

In one embodiment, the *Aspergillus oryzae* CJI1100 strain of the present application contains a DNA sequence encoding the 18S rRNA sequence of SEQ ID NO: 1.

In one embodiment, the *Aspergillus oryzae* CJI1100 strain of the present application does not produce aflatoxin.

Another aspect of the present application provides mycelium produced from the *Aspergillus oryzae* CJI1100 strain.

In the present application, "mycelium" refers collectively to a state in which hypha of fungi grow in a densely entangled state. The mycelium is a vegetative organ of fungi, and the mycelium produced by fungi has a high protein content.

In one embodiment, the mycelium has an amino acid composition comprising methionine.

In one embodiment, the mycelium contains methionine, and has an amino acid composition further comprises at least one amino acid selected from the group consisting of lysine, threonine, valine, isoleucine, leucine, phenylalanine, tryptophan and histidine.

In one embodiment, the mycelium has an amino acid composition comprising methionine, and further comprising two or more, three or more, four or more, five or more, six or more, seven or more, or eight or more amino acids selected from the group consisting of lysine, threonine, valine, isoleucine, leucine, phenylalanine, tryptophan and histidine.

In one embodiment, the mycelium has an amino acid composition comprising methionine, lysine, threonine, valine, isoleucine, leucine, phenylalanine, tryptophan and histidine.

In one embodiment, the mycelium the mycelium has a methionine(Met) content of 0.4g or more, 0.5g or more, 0.6g or more, 0.7g or more, 0.8g or more, 0.9g or more, 1g or more, 1.1g or more, or 1.2g or more based on 100 g of dry protein.

In one embodiment, the mycelium has an isoleucine(IIe) content of 3.5g or more, 3.6g or more, 3.7g or more, or 3.8g or more based on 100 g of dry protein.

In one embodiment, the mycelium has a glycine(Gly) content of 4g or more, 4.1g or more, 4.2g or more, or 4.3g or more based on 100 g of dry protein.

In one embodiment, the mycelium has at least one amino acid content selected from the group consisting of a valine content of 5g or more or 6g or more, a methionine content of 0.5g or more or 1g or more, an isoleucine content of 2.5g or more or 3.5g or more, a leucine content of 5g or more or 6g or more, a phenylalanine content of 3g or more or 4g or more, a histidine content of 1.6g or more or 1.8g or more, a tryptophan content of 2g or more or 2.3g or more and a threonine content of 3.5g or more or 4g or more, based on 100 g of dry protein.

In one embodiment, the mycelium has a glutamic acid(Glu) content of 10g or more, 11g or more, 12g or more, 13g or more, 13.1g or more, or 13.2g or more based on 100 g of dry protein.

In one embodiment, the dry protein content in the dried mycelium product may be 30 wt% to 70 wt%, specifically, 35 wt% to 70 wt%, 40 wt% to 60 wt% based on the total dry product weight.

In one embodiment, the mycelium does not contain aflatoxin, a fungal toxin.

Another aspect of the present application provides a method for producing mycelium comprising the following steps of: producing mycelium by culturing *Aspergillus oryzae* CJI1100 strain in a medium; and recovering the mycelium.

The step of culturing the *Aspergillus oryzae* CJI1100 strain to produce mycelium may comprise an inoculum preparation step of isolating the *Aspergillus oryzae* CIJ1100 strain or culturing spores to obtain a seed stock, and then inoculating the seed stock in an appropriate medium, culturing and proliferating thereof, and a main culture step to grow the desired mycelium from the inoculum in a liquid medium in large quantities.

The seed stock may be a culture of a fruit body tissue or spores of the CJI1100 strain.

The seed stock may be cultured and propagated in an appropriate medium to obtain inoculum.

In the inoculum preparation step, a nutrient medium such as potato dextrose agar medium and yeast malt glucose medium may be used as a culture medium for culturing the strain of the present application.

By preparing an inoculum and main culturing it, a large amount of spawn can be prepared using pure cultured bacteria under microbiologically stable conditions.

The main culture step is a step of growing mycelium in large quantities in a medium from an inoculum.

The main culture can be conducted using a solid medium or a liquid medium.

Culturing fungi in a liquid medium has the advantages of low cost, relatively safe from propagation of various bacteria, easy process control, and easy recovery of extracellular enzymes, mycelium and spores.

As a liquid medium usable in the present application, any medium that can be used for culturing mycelium used for food can be used without limitation, and for example, PDB medium or an equivalent component thereof can be used.

As a carbon source for the liquid medium, any one that is generally used for mycelium culture can be used without particular limitation. For example, monosaccharides, disaccharides, polysaccharides higher than trisaccharides, starch, and mixed saccharides such as waste molasses generated as a by-product during sucrose production can be used. Preferably, glucose, maltose, laminaribiose, potato starch, and corn starch may be used.

As a nitrogen source of the liquid medium, components generally added during liquid culture of fungi can be used, and for example, ammonium sulfate, ammonium phosphate, ammonium carbonate, ammonium acetate, peptone, yeast extract, corn steep liquor, casein hydrolysate, Wheat bran, meat extract and the like can be. In addition, inorganic salts such as potassium salts, magnesium salts, sodium salts, phosphates, manganese salts, iron salts, and zinc salts may be added to the liquid medium, and vitamins or amino acids may be further added thereto.

Shaking culture or liquid medium agitation may be performed during the main culture process. The agitation can be performed by rotating the agitator in the culture vessel, and the rotational speed of the agitator can be adjusted in an appropriate range, for example, in the range of 1 rpm to 300 rpm.

In addition, filtered air or compressed air can be blown into the culture vessel containing the liquid medium, thereby uniforming the contact between the liquid medium and the fungus mycelium, and increasing the concentration of oxygen, which is likely to be insufficient in a stationary liquid state.

The incubation temperature may be a temperature generally selected for culturing fungi and is not particularly limited, but, it may be appropriately selected in the temperature range of, for example, 5°C to 45°C, 10°C to 40°C, 20°C to 40°C, 25°C to 35°C.

The pH of the medium can be selected from the general pH range of the fungal culture medium, and can be adjusted to an appropriate pH range, for example within the pH range of 3 to 8.

The culture time can be adjusted to the time that mycelium can be obtained to the maximum amount, and the culture may be performed, for example, for 1 day to 30 days, for 1 day to 25 days, for 1 day to 20 days, for 1 day to 15 days, for 1 day to 10 days and for 1 day to 5 days.

In the step of recovering the mycelium, the method of recovering the fungus from the culture medium is not particularly limited, and known separation methods such as centrifugation, filtration separation, compression separation, slide culture separation and the like may be appropriately used.

The recovered mycelium can be used as a meat substitute by itself or after drying. For example, after adjusting the moisture content of the recovered mycelium to 30% to 90%, 40% to 80%, 50% to 70%, and 65% to 70%, the mycelium can be prepared directly as a meat substitute by adding and mixing a small amount of seasoning, minerals, protein supplements, or binding materials, or the recovered mycelium can also be used after drying. The drying method is not particularly limited and a known method such as natural drying, hot air drying, cold air drying, vacuum drying, or freeze drying may be used.

The recovered mycelium may be processed before use. The processing method of mycelium is not particularly limited, but, for example, an extrusion molding method may be used.

In the case of extrusion molding of mycelium, the manufacturing method may further comprise a step of extrusion molding of mycelium. During extrusion molding, the composition ratio of raw materials, input amount of raw materials, moisture content, screw rotation speed, and barrel heating temperature can be adjusted. During extrusion molding, the moisture content of the raw material can be adjusted to an appropriate range, and for example, the extrusion molding can be conducted by increasing the moisture content to about 30% to 40. In addition, it is possible to select an appropriate temperature range and pressure range during extrusion molding, and for example, the extrusion molding can be conducted at a pressure of 100 psi to 1000 psi under a temperature condition of 100°C to 170°C. A cooling die or a circular die can be used for the organization of the mycelium into proteins.

Another aspect of the present application provides a food containing the mycelium.

The food may be, for example, meat, meat substitute as vegetarian meat, processed meat products obtained by processing the meat or meat substitute, fungal protein (mycoprotein), flavoring agent or protein supplements, but is not limited thereto.

The meat substitute may be prepared by adding and mixing minerals, protein supplements or binding materials to the mycelium prepared by the above manufacturing method. The minerals are preferably calcium, magnesium, phosphate, and the like. As the protein supplements, materials of conventional meat substitutes such as hydrolyzed vegetable protein (HVP), for example, soybean protein and grain protein may be used, and meat such as beef or chicken, fish, vegetables, nuts, etc also may be used. As the binding material, egg albumin or the like may be used. In addition to the protein supplement and the binding material, calcium, glucan, nucleotide, sulfur-containing amino acid, glutamic acid, starch, fat, dietary fiber and the like may be mixed, and in addition, known minor ingredients such as flavoring and coloring agent may be mixed.

The meat may be prepared by a known method for producing meat by using the mycelium prepared by the above manufacturing method.

The processed meat products may be hams, sausages, bacons, dried stored meats, seasoned meats, processed meat extracts, meat-containing processed products, and packaged meat processed with meat or meat substitute.

The flavoring agent may be prepared by a known method for producing flavoring agent by using the mycelium prepared by the above manufacturing method.

The protein supplement can be prepared by a known method for producing protein supplements by using the mycelium prepared by the above manufacturing method.

Another aspect of the present application provides an inoculum composition comprising *Aspergillus oryzae* CJI1100 strain or its culture.

As used herein, the term "inoculum composition" refers to a preparation or composition artificially applied to a culture object for the start of the main culture, and may include a microorganism to be cultured and a component capable of providing essential components for the growth of the microorganism. The microorganism included in the inoculum composition may be a microorganism that predominantly grows in the culture object.

In the inoculum composition of the present application, the *Aspergillus oryzae* CJI1100 strain may be included in the form of shredded cell walls, viable cells, spores, dried bacteria or freeze-dried bacteria of the strain.

In one embodiment, the inoculum composition may contain spores of the *Aspergillus oryzae* CJI1100 strain at a concentration of 1x 10² spores/ml or more, specifically, a concentration of 1 × 10² to 1 × 10¹⁵ spores/ml, a concentration of 1× 10² to 1x 10¹³ spores/ml, a concentration of 1× 10² to 1× 10¹² spores/ml, a concentration of 1 × 10³ to 10¹⁰ spores/ml or a concentration of 1 × 10³-10⁹ spores/ml, but is not limited thereto. The concentration of the spores may vary depending on the use and formulation of the composition.

The culture of the strain refers to the result of inoculating and culturing the *Aspergillus oryzae* CJI1100 strain of the present application in a medium. Specifically, the culture of the present application may include a culture itself obtained by culturing the strain of the present application in a medium, a filtrate obtained by removing the strain by filtering or centrifuging the culture, or a concentrate thereof. In addition, the culture of the present application may be liquid or powder, and the powder may include powdered product after drying or lyophilizing the culture. The culture itself may include the *Aspergillus oryzae* CJI1100 strain of the present application itself, its spores, its metabolites, its mycelium, or its medium.

As the medium, a known fungal medium, such as potato dextrose agar medium or tryptic soy agar medium, may be used without limitation, and the medium may further contain auxiliary ingredients such as vitamins. Since the medium is the same as the contents of the medium described while explaining the culturing step of the method for producing mycelium from the *Aspergillus oryzae* CJI1100 strain, the above contents are invoked and will not be repeatedly described.

The inoculum composition of the present application may be in liquid or powder form. When distributed in the form of powder, it has the advantage of being easy to supply, maintain quality, and use. When the inoculum composition is formulated in the form of powder, granules, tablets or capsules, it may further include suitable carriers, cryoprotectants, excipients and diluents commonly used in food production.

According to one embodiment of the present application, the inoculum composition of the present application may further include a cryoprotectant, and more specifically, may further include at least one cryoprotectants selected from the group consisting of glycerol, trehalose, maltodextrin, skim milk powder, and starch. The cryoprotectant of the present application may be contained in an amount of 0.01 wt% to 20 wt%, 0.01 wt% to 10 wt%, based on the total weight of the inoculum of the present application. Specifically, the glycerol may be contained in an amount of 5 wt% to 20 wt%, the trehalose may be contained in an amount of 2 wt% to 10 wt%, the maltodextrin may be contained in an amount of 2 wt% to 10 wt%, the skim milk powder may be contained in an amount of 0.5 wt% to 2 wt%, and the starch may be contained in an amount of 0.1 wt% to 1 wt% in the inoculum composition.

The inoculum of the present application may further include an excipient. Specifically, the excipient may be at least one selected from the group consisting of glucose, dextrin, and skim milk. More specifically, the excipient may be contained in an amount of 75 wt% to 95 wt% or 85 wt% to 95 wt%, based on the total weight of the inoculum of the present application.

Another aspect of the present application may provide a food prepared using the inoculum composition. The food may contain the *Aspergillus oryzae* CJI1100 strain included in the inoculum composition or the mycelium produced from the strain. Since the food is the same as the contents of the medium described while explaining the food containing the mycelium produced from the *Aspergillus oryzae* CJI1100 strain, the above contents are invoked and will not be repeatedly described.

The novel *Aspergillus oryzae* CJI1100 strain of the present application can produce mycelium containing 9 kinds of essential amino acid. Therefore, mycelium containing all essential amino acids can be mass-produced by culturing the strain. In addition, the mycelium obtained by culturing the strain can be used as a variety of food materials including meat substitutes or alternative protein materials.

Further, since the mycelium produced by the strain of the present application is rich in glutamic acid, which gives a savory taste, it is effective to reduce the amount of conventional vegetable protein or chemical seasoning used in manufacturing meat substitutes.

However, the effects of the present application are not limited to the effects mentioned above, and other effects not mentioned will be clearly understood by those skilled in the art from the following description.

### BRIEF DESCRIPTION OF THE DRAWINGS

Fig. 1 shows the results of 18s rRNA sequence analysis of the *Aspergillus oryzae* CJI1100 strain of the present application. In Fig. 1, as a sequencing primer and a PCR primer, the nucleotide sequence of NS1 is shown as SEQ ID NO: 2, and the nucleotide sequence of NS8 is shown as SEQ ID NO: 3, respectively.
Fig. 2 shows the result of sequence analysis of the *NorBCypA* gene of the aflatoxin biosynthetic gene group of the *Aspergillus oryzae* CJI1100 strain of the present application. In Fig. 2, Contig Sequence is represented by SEQ ID NO: 4.
Fig. 3 shows the results of sequence analysis of the *aflR* gene of the aflatoxin biosynthetic gene group of the *Aspergillus oryzae* CJI1100 strain of the present application. In Fig. 3, Contig Sequence is represented by SEQ ID NO: 5.

### DETAILED DESCRIPTION

Hereinafter, the present application will be described in detail by Examples.

However, the following Examples specifically illustrate the present application, and the contents of the present application are no limited by the following Examples.

### [Example 1] Isolation and identification of Aspergillus oryzae strain

The fungal strain of the present application was isolated from traditional fermented soybean lumps. The fungal strain was isolated using potato dextrose agar (PDA, Difco) supplemented with 20 µg/ml chloramphenicol as a medium for isolation of fungi.

Molecular biological identification of the isolated fungal strain was analyzed by 18S rRNA analysis and sequence homology between *NorBCypA* gene and *aflR* gene, which are aflatoxin producing genes.

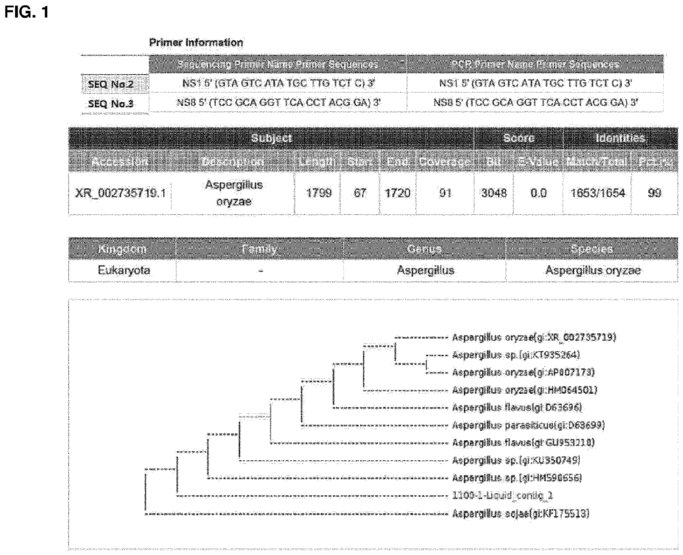

As shown in Fig. 1, as a result of 18S rRNA analysis, it was confirmed that the isolated stain was 99% identical to *Aspergillus oryzae.*

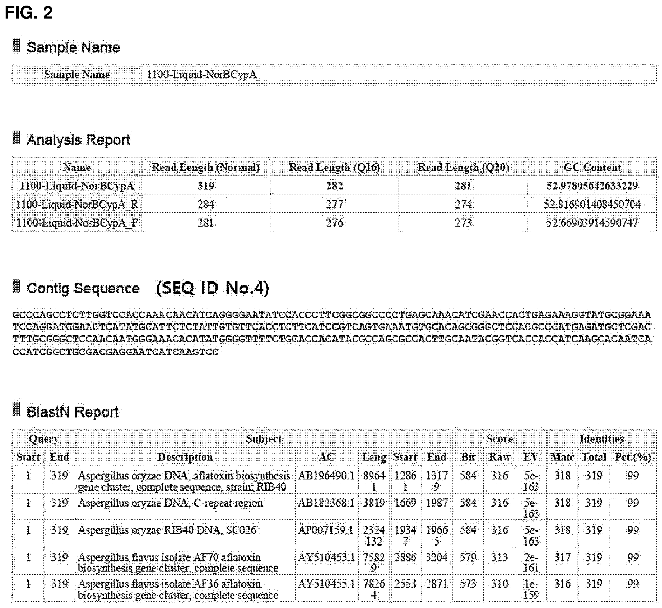

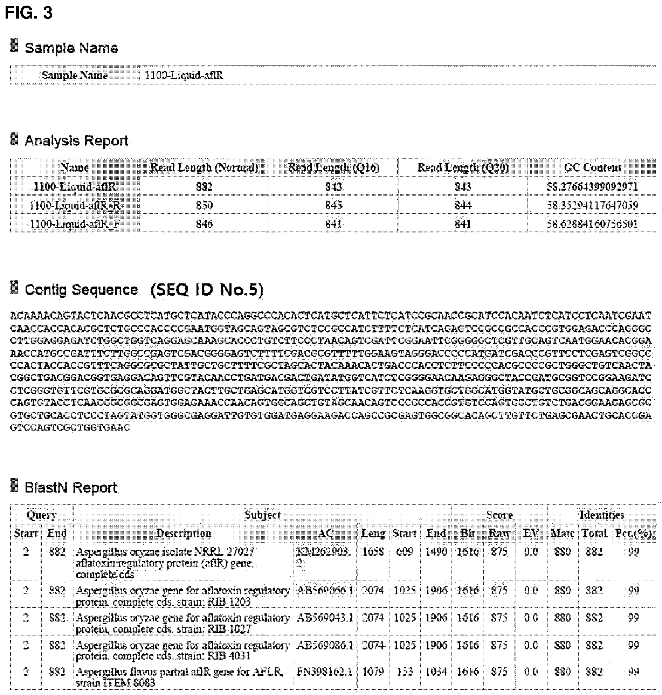

Further, as shown in Fig. 2 and Fig. 3, as a result of sequence analysis of *NorBCypA* gene and *aflR* gene of the aflatoxin biosynthetic gene group of the isolated strain, it was confirmed that the strain was consistent with *Aspergillus oryzae,* not *Aspergillus flavus,* which is known to produce aflatoxin. In addition, the length of the *NorBCypA* gene is 800 bp in *Aspergillus flavus,* a strain that produces aflatoxin, but the length was 300 to 400 bp in *Aspergillus oryzae.* It was confirmed that the *NorBCypA* of the *Aspergillus oryzae* CJI1100 strain of the present application is 319bp in length. Therefore, it was confirmed that the aflatoxin biosynthetic gene group in the fungal strain of the present application does not have a complete structure capable of producing aflatoxin.

As a result of the identification, the fungal strain of the present application was named *Aspergillus oryzae* CJI1100 strain of the genus *Aspergillus.* The strain was deposited at the Korean Culture Center of Microorganisms (KCCM) on October 05, 2021, and was given an accession number as KCCM13053P.

### [Example 2] Safety review of Aspergillus oryzae strain

From the above molecular biological identification results, it was confirmed that the fungal strain of the present application is *Aspergillus oryzae* having an aflatoxin biosynthetic gene group that cannot produce aflatoxin. However, in order to secure safety, it was verified again through the actual production of aflatoxin. The spores of *Aspergillus oryzae* CJI1100 strain preserved in glycerol stock at -80°C were spread on PDA medium and incubated at 30°C for 5 days. Then, the spores were recovered by washing with saline solution containing 0.05% Tween 80. Soybeans were prepared by sterilizing at 121°C for 15 minutes, inoculated with spores, and then incubated at 30°C for 5 days. The soybean culture obtained in this way was dried at 45°C for 48 hours and then pulverized to prepare an analysis sample. After extracting and purifying aflatoxin from the analysis sample, the total aflatoxin content was measured through liquid chromatography.

As a result of measuring total aflatoxin to examine the safety of the strain, it was confirmed that the *Aspergillus oryzae* CJ11100 strain of the present application does not produce a mycotoxin because aflatoxin was not detected.

**[Table 1]**

| Test Item | Test Result |
|---|---|
| Total Aflatoxin (*µ*g/kg) | Undetected |

### [Example 3] Comparative experiment of liquid cultured fungal mycelium

### [3-1] Preparation of inoculum

The spores of *Aspergillus oryzae* CJI1100 strain and strains owned by our company (CJI1130, CJI1140, CJI1333, CJT1105, CJI1126, CJT1050) preserved in glycerol stock at -80°C were spread on PDA medium, respectively, and incubated at 30°C for 5 days. Then, the spores were recovered with saline solution containing 0.05% Tween 80. In addition, as a comparative strain, *Fusarium Venenatum* ATCC20334 strain, which produces fungus mycelium, was used. The recovered spore solution was appropriately diluted and spores were counted through a microscope.

### [3-2] Main culture

Liquid culture of fungi was carried out using a 500 ml Erlenmeyer flask. For liquid culture of fungi, 200 ml of potato dextrose medium (PDB, Difco) was put in a 500 ml Erlenmeyer flask, and then sterilized at 121°C for 15 minutes. The fungal spores of each strain counted in Example [3-1] were inoculated into the PDB medium to a concentration of 1 × 10⁴ to 10⁵ spores/ml, and cultured at 30°C and 120 rpm for 5 days. After filtering the culture solution of fungi with a filter pack (Nasco), the produced mycelium was recovered and washed three times with saline solution to obtain fungal mycelium from which medium-derived components were removed.

### [3-3] Measurement of protein content of mycelium

Total nitrogen content was measured to determine protein content. Total nitrogen content was measured using the Kjeldahl Method. When organic nitrogen is decomposed into ammonium salt by adding sulfuric acid to the fungus mycelium obtained in Example 3-2 and concentrating by heating, an alkaline solution is added thereto and free NH₃ is collected in an acidic solution. Total nitrogen content was measured by titrating the NH₃ absorbed in the acidic solution. The measured total nitrogen value was multiplied by the nitrogen factor of 6.25 to calculate the crude protein content.

The protein content of mycelium produced by the *Aspergillus oryzae* CJ11100 strain of the present application and six *Aspergillus oryzae* comparative strains owned by our company (CJI1130, CJ11140, CJI1333, CJT1105, CJI1126, CJT1050) and the comparative strain *Fusarium Venenatum* ATCC20334 was evaluated. The results are shown in Table 2.

**[Table 2]**

| Strain | Dry Crude Protein Content (wt%) |
|---|---|
| *Aspergillus oryzae* CJI1130 | 44.6 |
| *Aspergillus oryzae* CJI1140 | 49.4 |
| *Aspergillus oryzae* CJI1333 | 56.3 |
| *Aspergillus oryzae* CJT1105 | 43.6 |
| *Aspergillus oryzae* CJI1100 | 40.1 |
| *Aspergillus oryzae* CJI1126 | 46.0 |
| *Aspergillus oryzae* CJT1050 | 47.2 |
| *Fusarium Venenatum* ATCC20334 | 58.7 |

As a result of the measurement, it was confirmed that the dry product of the mycelium produced by the fungal strain contains about 40 wt% to60 wt% of protein.

### [3-4] Measurement of amino acid composition of mycelium

After acid hydrolysis of the recovered fungus mycelium, the amino acid composition of the mycelium was measured using an amino acid analyzer. The measured amino acid value was converted into g/dry protein 100g and the contents were compared.

The amino acid compositions of the mycelium produced by 6 kinds of *Aspergillus oryzae* comparative strain (CJI1130, CJ11140, CJI1333, CJT1105, CJI1126, CJT1050) congenerous and homogeneous to the *Aspergillus oryzae* CJI1100 strain of the present application and comparative strain, *Fusarium Venenatum* ATCC20334 strain, were evaluated.

As shown in Table 3, it was confirmed that the mycelium produced by the CJI1100 strain of the present application has an amino acid composition containing all essential amino acids. When compared to the six *Aspergillus oryzae* comparative strains, it was confirmed that only the mycelium of *Aspergillus oryzae* CJI1100 strain contained methionine, an essential amino acid. When compared to the *Fusarium Venenatum* ATCC20334, it was also confirmed that the contents of essential amino acids, valine, methionine, isoleucine, leucine, phenylalanine, histidine, tryptophan, and threonine were higher in the mycelium produced from *Aspergillus oryzae* CJI1100 strain.

**[Table 3]**

| g/100g Dry protein | *A. oryzae* CJI1130 | *A. oryzae* CJI1140 | *A. oryzae* CJI1333 | *A. oryzae* CJT1105 | *A. oryzae* CJI1100 | *A. oryzae* CJI1126 | *A. oryzae* CJT1050 | *F. venenatum* ATCC20334 |
|---|---|---|---|---|---|---|---|---|
| Asp | 7.1 | 6.3 | 2.9 | 6.9 | 7.9 | 5.9 | 6.5 | 6.1 |
| Thr | 3.2 | 0.0 | 0.0 | 4.6 | 4.6 | 3.6 | 3.3 | 3.5 |
| Ser | 2.6 | 0.0 | 0.0 | 5.4 | 3.6 | 3.5 | 3.3 | 3.3 |
| Glu | 13.0 | 13.6 | 6.7 | 11.6 | 13.2 | 11.2 | 11.5 | 9.3 |
| Gly | 4.0 | 3.9 | 3.0 | 3.5 | 4.3 | 2.7 | 3.0 | 2.9 |
| Ala | 5.7 | 4.6 | 4.0 | 6.3 | 6.0 | 3.6 | 4.1 | 3.4 |
| Cys | 0.0 | 0.0 | 0.0 | 0.0 | 0.0 | 0.0 | 0.0 | 0.0 |
| Val | 6.6 | 4.6 | 5.1 | 5.5 | 6.4 | 3.9 | 4.1 | 3.2 |
| Met | 0.0 | 0.0 | 0.0 | 0.0 | 1.2 | 0.0 | 0.0 | 0.2 |
| Ile | 3.5 | 2.2 | 2.1 | 2.8 | 3.8 | 2.9 | 2.8 | 2.0 |
| Leu | 7.8 | 6.1 | 6.1 | 7.3 | 6.4 | 5.9 | 5.2 | 4.4 |
| Tyr | 0.0 | 0.0 | 0.0 | 0.0 | 2.3 | 0.0 | 0.0 | 2.6 |
| Phe | 7.7 | 4.6 | 5.7 | 9.4 | 4.2 | 5.9 | 5.7 | 2.4 |
| Lys | 5.1 | 4.6 | 3.4 | 5.6 | 5.4 | 3.9 | 3.8 | 5.1 |
| His | 2.3 | 1.8 | 0.9 | 3.5 | 2.1 | 0.9 | 2.0 | 1.5 |
| Arg | 4.0 | 1.9 | 2.0 | 3.0 | 4.0 | 3.1 | 3.0 | 3.6 |
| Pro | 0.0 | 0.0 | 0.0 | 0.0 | 0.0 | 0.0 | 0.0 | 2.4 |
| Trp | 3.4 | 3.0 | 2.4 | 2.8 | 2.5 | 2.2 | 2.2 | 0.5 |

In addition, to evaluate protein quality, the amino acid score (AAS) value was calculated by dividing the content (mg) of each essential amino acid per gram of fungal mycelium protein by the content (mg) of the same essential amino acid per gram of reference protein. The amino acid score was calculated by the formula AAS= (mg of AA in 1g protein) / (mg of AA in requirement pattern). The content (mg) of each essential amino acid per gram of protein in the reference protein is called the amino acid requirement pattern, and the reference protein may be egg protein, and the content of essential amino acid per gram of reference protein may use the value suggested by WHO. The WHO 2007 standard was referred to the amino acid requirement pattern. The amino acid score value is based on 1.0, and an amino acid less than 1.0 is defined as a limiting amino acid.

The results of calculating the amino acid score of the mycelium produced by the *Aspergillus oryzae* CJI1100 and *Fusarium Venenatum* ATCC20334 are shown in Table 4 below.

**[Table 4]**

| Amino Acid | *Aspergillus oryzae* CJI1100 Amino Acid Score | *Fusarium Venenatum* ATCC20334 Amino Acid Score | Amino Acid Requirement Pattern *WHO 2007 (mg/g) |
|---|---|---|---|
| His | 1.2 | 0.8 | 18 |
| Ile | 1.2 | 0.6 | 31 |
| Leu | 1.0 | 0.7 | 63 |
| Lys | 1.0 | 1.0 | 52 |
| Met+Cys | 0.5 | 0.1 | 26 |
| Phe+Tyr | 1.4 | 1.1 | 46 |
| Thr | 1.7 | 1.3 | 27 |
| Trp | 3.3 | 0.7 | 7.4 |
| Val | 1.5 | 0.8 | 42 |

It was found that among the amino acid compositions of the mycelium produced by *Aspergillus oryzae* CJI1100 strain, methionine and cytosine (Met+Cys) are limiting amino acids because their amino acid score values are less than 1.0, but the amino acid scores of all essential amino acids except for these amino acids exceed 1.0, so they meet the criteria. Since the mycelium produced from *Aspergillus oryzae* CJI1100 strain contains all kinds of essential amino acids, it can be improved as a complete protein through optimization of medium and culture conditions.

### [Example 4] Extrusion molding of mycelium

After adjusting the moisture content so that the moisture content of the mycelium of the strain of the present application obtained in Example 3-2 is 40%, extrusion molding was performed using a cooling die at a pressure of 100 to 1000 psi under a temperature condition of 100 to170°C to prepare mycelium that can be used for food.

### [Example 5] Manufacture of meat substitute using mycelium

A meat substitute was prepared by adding a small amount of salt, sugar, pepper, rice flour and the like to the mycelium of the present application.

Specifically, after adjusting the moisture content so that the moisture content of the mycelium of the strain of the present application obtained in Example 3-2 is 65 to 70%, salt 0.81%, black pepper 0.1%, garlic powder 0.51%, white sugar 1.52%, canola oil 0.81%, meat flavor 0.3%, and rice flour 5.87% were added thereto to prepare a meat substitute.

### [Example 6] Manufacture of bacon using mycelium

Bacon was prepared using the meat substitute prepared in Example 5 above. Specifically, the prepared meat substitute was molded into a wide plate shape with a thickness of 2 inches. Then, it was sliced thinly to a size that was easy to process with bacon. The sliced mycelium pieces were pickled in salt solution with a salinity of 1 to 5%, then colored with salt, sugar, coconut oil, and beet juice, and liquid smoke was added to prepare smokey and savory bacon.

### [Example 7] Manufacture of mycoprotein

It was carried out according to the common vegetable protein manufacturing method (Korea Food Research Institute, Development of new functional food materials using higher fungi and application research, Final research report, Ministry of Agriculture and Forestry, November 18, 2002). After mixing the mycelium prepared in Example 3-2 above, corn hull and egg white at the ratio of 40%, 30% and 30%, extrusion molding was conducted using a cooling die in the same way as in Example 4 above to obtain mycoprotein.

### [Example 8] Manufacture of meat substitute using mycoprotein

In addition, a meat substitute was manufactured according to a conventional meat substitute (artificial meat) manufacturing method in which starch, water, gum, emulsifier and the like were added to the protein material of the mycoprotein of the present application and extrusion molding was conducted at high temperature and high pressure to obtain a fibrous tissue. Specifically, a meat substitute was prepared by adding 50 wt% of the mycoprotein of the present application prepared in Example 5 and adding a small amount of seasoning, spice, coloring and the like.

### [Example 9] Manufacture of natural meat flavoring agent from mycelium

The mycelium prepared in Example 3-2 was dried and powdered, and the anchovy with the intestines removed, the washed and cut kelp and seaweed were prepared. 20 wt% of anchovy, 20 wt% of kelp and 20 wt% of seaweed were added to 100 wt% of water, and heated at 100°C until the amount of water was reduced to 1/2 to obtain a hot water extract. After immersing anchovy, kelp, and seaweed in the hot water extract for 12 hours, they were dried and powdered. 65 wt% of the powdered mycelium, 10 wt% of anchovy powder, 10 wt% of kelp powder, and 5 wt% of seaweed powder were mixed to prepare a natural meat flavoring agent.

In the above, representative examples of the present application have been exemplarily described, but the scope of the present application is not limited to the specific example as described above, and those skilled in the art will be able to make appropriate changes within the scope described in the claims of the present application.
Name of depository : Korean Culture Center of Microorganisms
Accession Number: KCCM13053P
Accession Date : 20211005

## Claims

1. *Aspergillus oryzae* CJI1100 strain (Accession Number: KCCM13053P) that produces mycelium having an amino acid composition comprising methionine.

2. The *Aspergillus oryzae* CJI1100 strain (Accession Number: KCCM13053P) of claim 1, wherein the *Aspergillus oryzae* CJI1100 strain contains a DNA sequence encoding the 18S rRNA sequence of SEQ ID NO: 1.

3. The *Aspergillus oryzae* CJI1100 strain (Accession Number: KCCM13053P) of claim 1, wherein the amino acid composition further comprises at least one amino acid selected from the group consisting of lysine, threonine, valine, isoleucine, leucine, phenylalanine, tryptophan and histidine.

4. The *Aspergillus oryzae* CJI1100 strain (Accession Number: KCCM13053P) of claim 1, wherein the *Aspergillus oryzae* CJI1100 strain does not produce aflatoxin.

5. Mycelium produced from the *Aspergillus oryzae* CJI1100 strain (Accession Number: KCCM13053P) of any one of claim 1 to claim 4.

6. The mycelium of claim 5, wherein the mycelium has an amino acid composition comprising methionine.

7. The mycelium of claim 6, wherein the amino acid composition further comprises at least one amino acid selected from the group consisting of lysine, threonine, valine, isoleucine, leucine, phenylalanine, tryptophan and histidine.

8. The mycelium of claim 5, wherein the mycelium does not contain aflatoxin.

9. The mycelium of claim 5, wherein the protein content in the dried mycelium product is 30 wt% to 70 wt% by weight based on the total dry product weight.

10. A method for producing mycelium comprising the following steps of: producing mycelium by culturing *Aspergillus oryzae* CJI1100 strain (Accession Number: KCCM13053P) in a medium; and recovering the produced mycelium.

11. The method for producing mycelium of claim 10, wherein the step of recovering the mycelium comprises a step of filtering the culture solution of the strain.

12. The method for producing mycelium of claim 10, which further comprises a step of extruding and molding the recovered mycelium.

13. A food containing the mycelium produced from the *Aspergillus oryzae* CJ11100 strain of any one of claim 1 to claim 4.

14. The food of claim 13, wherein the food is a meat substitute, a meat product processed from the meat substitute, a fungal protein(mycoprotein), a flavoring agent or a protein supplements.

15. An inoculum composition comprising *Aspergillus oryzae* CJI1100 strain (Accession Number: KCCM13053P) or its culture.

16. The inoculum composition of claim 15, wherein the inoculums composition comprises spores of the *Aspergillus oryzae* CJI1100 strain (Accession Number: KCCM13053P) at a concentration of 10² spores/ml or more.

17. The inoculum composition of claim 15, wherein the inoculum composition further comprises at least one cryoprotectant selected from the group consisting of glycerol, trehalose, maltodextrin, skim milk powder and starch.
